**Europäisches Patentamt**

**European Patent Office**

**Office européen des brevets**

(11) Veröffentlichungsnummer: **0 070 466**
**A1**

## (12) EUROPÄISCHE PATENTANMELDUNG

(21) Anmeldenummer: **82106118.1**

(22) Anmeldetag: **08.07.82**

(51) Int. Cl.³: **C 12 Q 1/24**

(30) Priorität: **13.07.81 DE 3127654**

(43) Veröffentlichungstag der Anmeldung: **26.01.83**
Patentblatt 83/4

(84) Benannte Vertragsstaaten: **AT BE CH DE FR GB IT LI NL SE**

(71) Anmelder: **HOECHST AKTIENGESELLSCHAFT,**
**Postfach 80 03 20, D-6230 Frankfurt/Main 80 (DE)**

(72) Erfinder: **Nesemann, Georg, Dr., Bornstrasse 73,**
**D-6238 Hofheim am Taunus (DE)**
Erfinder: **Sittig, Wolfgang, Sulzbacher Weg 2,**
**D-6238 Hofheim am Taunus (DE)**
Erfinder: **Stanke, Georg, Breslauer Strasse 53,**
**D-6233 Kelkheim Taunus (DE)**

(54) **Automatisiertes Verfahren zur Selektion von Mikroorganismen.**

(57) Automatisiertes Verfahren zur Selektion von Mikroorganismen, bei dem man zur Erhöhung der Anzahl der pro Zeiteinheit ausgeprüften Mikroorganismen mehrere an sich bekannte Einzelschritte gemeinsam einsetzt, wobei

(a) unter sterilen Bedingungen mit einem Nährmedium angereicherte Agar-Blöckchen hergestellt werden;

(b) diese Agar-Blöckchen mit je einem Tropfen einer Sporensuspension geimpft werden, wobei durch fotometrische Kontrolle sichergestellt wird, daß in der Regel jeder Tropfen nur eine Spore enthält;

(c) die beimpften Agar-Blöckchen dann im Brutschrank unter Bedingungen aufbewahrt werden, die eine Koloniebildung aus den Sporen erlauben;

(d) das eine Sporenkolonie enthaltende Agar-Blöckchen dann auf einem mit Testkeimen vermischten Nährboden aufgesetzt und

(e) nach erneuter Aufbewahrung im Brutschrank aus Koloniedurchmesser und Hemmhofdurchmesser mit Hilfe eines Computers der Prozentindex errechnet und daraus ein Histogramm ausgedruckt wird.

## Automatisiertes Verfahren zur Selektion von Mikroorganismen

Gegenstand der Erfindung ist ein automatisiertes Verfahren zur Selektion von Mikroorganismen, bei dem man zur Erhöhung der Anzahl der pro Zeiteinheit ausgeprüften Mikroorganismen mehrere an sich bekannte Einzelschritte variiert und gemeinsam einsetzt.

Die Verbesserung der Eigenschaften von Mikroorganismen ist für die industrielle Mikrobiologie eine ganz entscheidende Aufgabe. Dies gilt insbesondere für die Ausbeutesteigerung bei der Antibiotika-Produktion, durch die es möglich geworden ist, die Herstellungspreise drastisch zu senken und Antibiotika für eine breite Anwendung zugänglich zu machen.

Bei der Verbesserung der Ausbeuten wird im wesentlichen so vorgegangen, daß man das genetische Material der betreffenden Stämme verändert, sei es durch Strahlen, durch mutagene Chemikalien oder durch Maßnahmen, die heute meist unter dem Namen "genetic engineering" zusammengefaßt werden. Dabei entstehen Stämme, die durch Veränderung ihrer DNA neue Eigenschaften aufweisen. Diese sind im allgemeinen, da sie mehr oder weniger richtungslos erfolgen, negativ im Sinne des gewünschten Effektes. Nur ganz selten, in der Größenordnung $10^{-5}$ bis $10^{-6}$, treten Verbesserungen in positiver Richtung auf. Um diese Stämme zu erkennen, bedarf es eines großen Arbeitsaufwandes, da Tausende von Stämmen auf ihre Antibiotika-Produktion überprüft werden müssen. Diese Überprüfung geschieht im allgemeinen durch Züchtung der Stämme in kleinen Kölbchen in Submers-Kulturen und Messung der gebildeten Antibiotika-Konzentrationen in den Kulturlösungen.

Mit dem Ziel, den Aufwand bei der Selektion zu verkleinern, wurde von Ichikawa und Mitarbeitern /T.Ichikawa, M. Pate und A. Ozak in "Folia Microbiologica" 16, 218-224 (1971)_7 die
Agar-Stück-Methode eingeführt. Dabei werden die Sporensuspensionen in hoher Verdünnung auf einen Agar-Nährboden plattiert, nach dem Anwachsen die einzelnen Kolonien mit dem umgebenden Agar mit einem Korkbohrer ausgestochen und einzeln bebrütet. Dabei wird der Nährstoff verbraucht und zum Teil in das Antibiotikum umgewandelt, welches sich im Agar ansammelt. Anschließend werden die Agar-Blöckchen auf einen mit einem Testkeim beimpften Nährboden gesetzt und anhand der entstehenden Hemmzone die gebildete Antibiotika-Menge bestimmt. Die besten Stämme werden so erkannt, abgeimpft und weitergezüchtet.

Diese Methode wurde nicht nur für Streptomyceten, sondern auch für Pilze angewendet /vgl. A. Trilli, V. Michelinie, V. Mantorani und J. Pirt, "Antimicrobial Agents and Chemoth.", 13, 7-13 (1978)_7. Verbessert wurde diese Methode, indem nicht nur die Hemmzone, sondern auch die Koloniegröße bestimmt und daraus der Potenz-Index gebildet wurde /C. Ball und M.P. Gronagle in "J. Appl. Bact.", 45, 67-74 (1978)_7.

Unabhängig davon sind Versuche durchgeführt worden, mikrobiologische Selektionsverfahren zu mechanisieren und zu automatisieren. Bekannt geworden ist der sogenannte "Dumbwaiter" (vgl. Glaser in "Fortune", 100-101, Februar 1974; D.A. Glaser in "New Approaches to the Identification of Microoganisms", edited by C.-G. Hedén, T. Illéni; Wiley, New York (1975), Seiten 3 - 12), bei dem mit Hilfe einer vibrierenden Düse die entsprechend verdünnte Suspension der Organismen in feine Tröpfchen verteilt, auf mit Nährboden beschickte Folien geimpft, nach Bildung der Kolonien fotografiert und dann mit einem Computer ausgewertet werden.

Diese vielfältigen Versuche zeigen, daß ein außerordentlich großes und dringendes Bedürfnis besteht, die bisher bekannten Selektionsverfahren zu verbessern. Das wird im übrigen auch dadurch unterstrichen, daß für bestimmte Teilschritte mikrobiologischer Verfahren bereits Einzelvorrichtungen entwickelt worden sind, die früher manuell durchgeführte Maßnahmen übernehmen konnten. Trotz aller Anstrengungen ist es jedoch bisher nicht gelungen, mikrobiologische Selektionsverfahren vollständig zu automatisieren.

Die vorliegende Erfindung betrifft nun ein neues Verfahren, bei dem einerseits die Agar-Stück-Methode durch Mechanisierung und Automatisierung der Arbeitsvorgänge so verändert wurde, daß deren Leistungsfähigkeit stark erhöht werden konnte, insbesondere dadurch, daß die Agarblöckchen durch Gießen hergestellt werden, und/oder andererseits durch fotometrische Methoden sichergestellt wird, daß bei der Feinverteilung der Organismensuspension in der Regel jeder Tropfen tatsächlich nur eine einzige Spore bzw. Zelle enthält. Damit sind die Chancen, einen verbesserten Stamm zu finden, wesentlich gestiegen.

Gegenstand der Erfindung ist somit ein automatisiertes Verfahren zur Selektion von Mikroorganismen, bei dem man zur Erhöhung der Anzahl der pro Zeiteinheit ausgeprüften Mikroorganismen mehrere an sich bekannte Einzelschritte gemeinsam einsetzt, wobei

(a) unter sterilen Bedingungen mit einem Nährmedium angereicherte Agar-Blöckchen hergestellt werden;

(b) diese Agar-Blöckchen mit je einem Tropfen einer Sporensuspension beimpft werden, wobei durch fotometrische Kontrolle sichergestellt wird, daß in der Regel jeder Tropfen nur eine Spore enthält;

(c) die beimpften Agar-Blöckchen dann im Brutschrank unter Bedingungen aufbewahrt werden, die eine Koloniebildung aus den Sporen erlauben;

(d) die eine Sporenkolonie enthaltenden Agar-Blöckchen dann auf einen mit Testkeimen vermischten Nährboden aufgesetzt und

(e) nach erneuter Aufbewahrung im Brutschrank aus Koloniedurchmesser und Hemmhofdurchmesser mit Hilfe eines Computers der Potenzindex errechnet und daraus eine Auswahl getroffen wird.

Im folgenden werden bevorzugte Ausgestaltungen der Erfindung näher erläutert.

Der erste Schritt des erfindungsgemäßen Verfahrens besteht in der automatisierten Herstellung der Agar-Blöckchen. Dazu werden Metalleisten, deren Enden so geformt sind, daß sie durch einen automatischen Greifer erfaßt werden können und die Vertiefungen zur Aufnahme und zum Transport von Röhrchen tragen, in einem Magazin übereinander gestapelt und sterilisiert. Dieses Magazin wird mit einer Maschine verbunden, die die jeweils unterste Transportleiste ergreift und sie taktweise herauszieht. Der Takt ist so eingestellt, daß in jeder Taktpause in eine Vertiefung der herausgezogenen Transportleiste ein Kunststoffröhrchen, das oben und unten offen ist, eingelegt wird. Das Röhrchen wird aus einem Vorratsbehälter durch Vibration herausbefördert und über eine Rutsche in einen Röhrchensetzer befördert. Jedes Röhrchen ist vor Versuchsbeginn sterilisiert worden und wird auf der Transportleiste steril in eine automatisch arbeitende Dosiervorrichtung befördert.

In der Dosiervorrichtung wird jedes Röhrchen mit 0,5 ml eines geeigneten Nährmediums gefüllt, das aus 1,5 bis 4 Gew.-% Agar und geeigneten Nährstoffen wie Kohlenhydraten, Proteinen, Nährsalzen, Spurenelementen und auch Vitaminen bestehen kann. Dieses Nährmedium wurde vorher in einem Vorratsbehälter sterilisiert und dann in einem Wasserbad warm gehalten (z.B. 55°C). Mittels einer Dosierpumpe wird das Nährmedium steril über beheizte Leitungen zum Abfüllstutzen befördert. Eine Fotozelle kontrolliert das Vorhandensein eines Röhrchens und verhindert die Nährmediumabgabe bei fehlendem Röhrchen.

Die Transportleiste läuft nach Füllung sämtlicher Röhrchen über eine Abkühlstrecke, um das Nährmedium erstarren zu lassen.

In den gefüllten Röhrchen sind jetzt kleine Agar-Blöckchen entstanden, die nun beimpft werden müssen. Dies erfolgt durch einen Tropfengenerator, bei dem ein Synchronmotor eine Sporensuspension durch eine Kanüle drückt. Durch einen Lautsprecher, der von einem Rechteckgenerator Impulse erhält, werden Tropfen abgerissen, deren Größe je nach Frequenz variiert. Bei geeigneter Einstellung der Sporendichte lassen sich so Tropfen herstellen, die zu 30 bis 40 % nur eine Spore enthalten. Diese Werte lassen sich wesentlich verbessern, wenn man das Auftreten von Sporen in der Kanüle mit einem Laserstrahl kontrolliert. Über einen Fotodetektor, elektronische Steuerung und Impulshöhenanalysator kann der Lautsprecher so gesteuert werden, daß sich jeweils nur eine Spore im Tropfen befindet. Die Beimpfung kann auch durch ein Durchflußcytofotometer erfolgen, z.B. durch das Gerät "Cytofluorograph System 50 H" der Fa. Ortho Instruments, das Gerät "FACS IV" der Fa. Becton Dickinson oder durch das Gerät "TPS I" der Fa. Coulter Electronics GmbH. Dabei können auch keimfähige von abgestorbenen Sporen getrennt und letztere verworfen werden, indem man z.B. eine Vitalfärbung mit fluoreszierenden Farbstoffen durchführt oder die Sporen auskeimen läßt und die unterschiedlichen Signale gekeimter und ungekeimter Sporen für die Trennung benutzt.

Die Transportleisten mit den beimpften Agar-Blöckchen werden anschließend maschinell gesammelt und mechanisch lagenweise in einen Brutschrank befördert.

Dieser Brutschrank ist mit Regalen zur Aufnahme der Transportleisten ausgestattet. Er wird über ein Wasserbad geheizt oder gekühlt, um immer eine gleichmäßige Temperatur und eine gleichmäßige Luftfeuchtigkeit sicherzustellen. Zusätzlich wird die Luft durch einen Ventilator umgewälzt und von Zeit zu Zeit durch eine Berieselungsanlage angefeuchtet. Diese Anlage entnimmt entsalztes Wasser aus dem Wasserbad und pumpt es über einen Sterilfilter durch Düsen auf eine Rieselwand, die sich im Brutschrank befindet. Die Rieselwand ist mit Öffnungen versehen, durch die die umgewälzte Luft geleitet wird. Durch die Kombination dieser Maßnahmen wird erreicht, daß die beimpften Agar-Blöckchen nicht austrocknen, sich aber auch kein Kondenswasser ansammelt. Die Agar-Blöckchen werden so im Brutschrank bei Temperaturen zwischen 15 und 45°C, vorzugsweise bei 20 bis 37°C und einer relativen Luftfeuchte von etwa 95 % für 1 bis 10 Tage, vorzugsweise für 3 bis 5 Tage aufbewahrt. Unter diesen Bedingungen kann ein gleichmäßiges Wachstum der verimpften Keime erfolgen.

Nachdem die Sporen zu Kolonien ausgewachsen sind und diese die zur Verfügung stehenden Nährstoffe aufgebraucht haben, wobei diese teilweise in die gewünschten Stoffwechselprodukte umgewandelt wurden, erfolgt die Ausprüfung der Leistungsfähigkeit der Kolonien durch quantitative Bestimmung der Stoffwechsel-Produkte.

Dazu wird der Brutschrank mit den auf den Agar-Blöckchen ausgewachsenen Kolonien an eine weitere automatisch arbeitende Maschine angeschlossen. Diese Maschine zieht zunächst mittels eines Greifers die Transportleisten mit den die Agar-Blöckchen enthaltenden Röhrchen aus dem Brutschrank heraus und führt sie einem Umsetzer zu. Der Umsetzer setzt die Röhrchen mit den Agar-Blöckchen unter Verdoppelung ihrer Abstände auf den vorher zubereiteten Nährboden einer Testkeimwanne.

Eine derartige Testkeimwanne besteht im allgemeinen aus Aluminium, das mit schwarzem Kunststoff überzogen ist. Ihre Abmessungen sind ausreichend groß, um gleichzeitig eine größere Anzahl von Agar-Blöckchen aufzunehmen, wobei für einen genügend großen Abstand zwischen den einzelnen Agar-Blöckchen gesorgt werden muß, damit sie sich gegenseitig nicht beeinflussen können.

Eine derartige Testwanne wird nun unter sterilen Bedingungen mit einem geeigneten Nährmedium gefüllt. Dieses Medium, das natürlich ebenfalls sterilisiert werden muß, enthält neben 1,2 bis 4 Gew.-% Agar je nach Testkeim Kohlenhydrate (z.B.

Zucker, Stärke oder höhere Alkohole), Stickstoffquellen
(z.B. Peptone, Bouillon, Fleischextrakt, Ammoniumsalze),
anorganische Salze und Spurenelemente sowie auch Vitamine
und andere übliche, den Test günstig beeinflussende Zusätze.

Das sterile Nährmedium wird dann auf etwa 50°C abgekühlt
und durch beheizte Leitungen in ein Mischgefäß gepumpt. Dort
befindet sich ein Niveauregler, der den Zulauf über eine
automatische Pumpe steuert. In einem anderen Gefäß befindet
sich eine vorgezüchtete Suspension von Testkeimen, z.B. von
Staphylokokken, die von dort mit einer ebenfalls von einem
Niveauregler gesteuerten Dosierpumpe in das Mischgefäß gepumpt und dort durch Verrührung mit dem Nährmedium homogen
gemischt wird. Das beimpfte Nährmedium wird dann automatisch
in die Testkeimwannen gefüllt, wobei 30 bis 80 ml, im allgemeinen 50 ml, pro Wanne eingefüllt werden. Hierzu dient eine
Dosierpumpe, die sich auf einem maschinell bewegten Tisch
befindet, der so gesteuert wird, daß der Abfüllstutzen nach
jeder Füllung zur nächsten Wanne weiterwandert, bis alle
Wannen des Brutschrankes gefüllt sind. Dann wird gekühlt,
bis das Nährmedium erstarrt ist.

Der mit den fertig vorbereiteten Testkeimwannen gefüllte
Brutschrank wird dann ebenfalls an die Maschine angeschlossen, die die beimpften Agar-Blöckchen heranführt. Durch
einen Greifer werden die Testkeimwannen jetzt lagenweise
herausgezogen und mechanisch einzeln mit den beimpften Agar-
Blöckchen besetzt. Dann werden die Testkeimwannen lagenweise
in den Brutschrank zurückgeführt.

Man läßt nun die beimpften Agar-Blöckchen 12 bis 48 Stunden,
vorzugsweise etwa 18 Stunden bei 20 bis 45°C auf den mit
den Testkeimen vermischten Nährboden einwirken. Dabei
diffundiert das in dem Nährboden der Agar-Blöckchen angesammelte Antibiotikum in das Testkeimmedium und führt dort
zur Bildung einer Hemmzone. Die Größe dieser Hemmzonen ist

0070466

ein Maß für die Menge des gebildeten Antibiotikums. Nach deutlicher Ausbildung der Hemmzonen werden aus dem Brutschrank die Testwannen wiederum automatisch entnommen und einer Kamera mit elektronischer Bildauswertung zur Messung von Koloniedurchmesser und Hemmhofdurchmesser zugeführt. Nach der Messung werden die Testkeimwannen in den Testbrutschrank zurückgeführt und dort zur Auswertung aufbewahrt.

Die Messung von Koloniedurchmesser und Hemmhofdurchmesser kann z.B. durch ein "Struktur-Analysen-System" ("texture analysing system" =TAS) der Fa. Leitz oder ein anderes gleichwertiges Gerät erfolgen. Dieses muß so mit dem Programm der Maschine synchronisiert sein, daß Ablösung und Vortransport der Testkeimwanne abwechselnd erfolgt. Die Anlage selbst kann ihre Ergebnisse zur Auswertung auf einen Computer, z.B. einen Olivetti P 6060, geben. Dieser ist so programmiert, daß er die gemessenen Werte numeriert, die Anzahl der Kolonien pro Röhrchen kontrolliert, aus Koloniedurchmesser und Hemmhofdurchmesser den Potenzindex errechnet, nach Abschluß der Messungen die Potenzindices klassifiziert, speichert und daraus eine Verteilungskurve (Histogramm) erstellt und ausdruckt. Anhand dieses Histogramms kann entschieden werden, welche Kolonien bestimmter Potenzindexklassen herausselektioniert werden sollen. Der Computer kann danach auch die Nummern selektionierter Kolonien ausdrucken.

Das hier geschilderte Verfahren kann nicht nur auf Antibiotika angewendet werden. Es ist auch zur Selektion von Mikroorganismen mit quantitativen Veränderungen in der Produktion anderer Stoffwechselprodukte, wie Antimetaboliten, Enzym-Inhibitoren, Wuchsstoffen oder Vitaminen geeignet. Voraussetzung dabei ist die Entwicklung eines geeignete gut ablesbaren Testsystems durch Verwendung geeigneter Mikroorganismen oder von Farb- oder Fällungsreaktionen, z.B. Immunpräzipitationen. Es kann aber auch zur Selektion von Organismen mit bestimmten Stoffwechselprodukten verwendet werden.

- 9 -

0070466

Die Anwendung ist außerdem auch nicht auf die Selektion von Mikroorganismen beschränkt, sondern kann auch zur Selektion von Zellkulturen Verwendung finden.

Patentansprüche:

1. Automatisiertes Verfahren zur Selektion von Mikroorganismen, dadurch gekennzeichnet, daß man zur Erhöhung der Anzahl der pro Zeiteinheit ausgeprüften Mikroorganismen mehrere an sich bekannte Einzelschritte gemeinsam einsetzt, wobei

   (a) unter sterilen Bedingungen mit einem Nährmedium angereicherte Agar-Blöckchen hergestellt werden;

   (b) diese Agar-Blöckchen mit je einem Tropfen einer Sporensuspension geimpft werden, wobei durch fotometrische Kontrolle sichergestellt wird, daß in der Regel jeder Tropfen nur eine Spore enthält;

   (c) die beimpften Agar-Blöckchen dann im Brutschrank unter Bedingungen aufbewahrt werden, die eine Koloniebildung aus den Sporen erlauben;

   (d) das eine Sporenkolonie enthaltende Agar-Blöckchen dann auf einen mit Testkeimen vermischten Nährboden aufgesetzt und

   (e) nach erneuter Aufbewahrung im Brutschrank aus Koloniedurchmesser und Hemmhofdurchmesser mit Hilfe eines Computers der Potenzindex errechnet und daraus eine Auswahl getorffen wird.

2. Verfahren nach Anspruch 1, dadurch gekennzeichnet, daß die Agar-Blöckchen in Röhrchen hergestellt werden, die auf einem als Leiste ausgebildeten Träger in eine automatisch arbeitende Dosiervorrichtung transportiert und dort mit einem Agar enthaltenden Nährmedium gefüllt werden.

3. Verfahren nach Anspruch 1, dadurch gekennzeichnet, daß die Agar-Blöckchen mit Einzelsporen in einem Durchflußcytofotometer unter gleichzeitiger Abtrennung toter Sporen geimpft werden.

0070466
HOE 81/F 168

4. Verfahren nach Anspruch 1, dadurch gekennzeichnet, daß die beimpften Agar-Blöckchen im Brutschrank bei Temperaturen zwischen 15 und 45°C, vorzugsweise 20 bis 37°C, und einer relativen Luftfeuchte von etwa 95 % für 1 bis 10 Tage, vorzugsweise für 3 bis 5 Tage, aufbewahrt werden.

5. Verfahren nach Anspruch 1, dadurch gekennzeichnet, daß man den mit Testkeimen vermischten Nährboden automatisch in eine Testwanne eindosiert, auf dem nach seinem Erstarren mechanisch die die Sporenkolonien enthaltenden Agar-Blöckchen in einem so großen Abstand gesetzt werden, daß sie sich gegenseitig nicht beeinflussen können.

6. Verfahren nach Anspruch 1, dadurch gekennzeichnet, daß man die die Sporenkolonien enthaltenden Agar-Blöckchen 12 bis 48 Stunden, vorzugsweise etwa 18 Stunden, bei 20 bis 45°C auf den mit Testkeimen vermischten Nährboden einwirken läßt.

7. Verfahren nach Anspruch 1, dadurch gekennzeichnet, daß nach dem Bebrüten die Testwanne automatisch dem Brutschrank entnommen und einer Kamera zugeführt wird, die mit einem Computer als "Struktur-Analysen-System" (= texture analysing system) verbunden ist.

8. Verfahren nach Anspruch 1, dadurch gekennzeichnet, daß der Computer aus Koloniedurchmesser und Hemmhofdurchmesser den Potenz-Index errechnet, die Ergebnisse klassifiziert, speichert und eine Verteilungskurve der Potenzindices ausdruckt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | C 12 Q    1/24 |
| A | DE-A-2 927 141   (EXNER, M.)<br>*Anspruch 1; Seiten 4-7* | 1,2 | |
| | --- | | |
| A | DE-B-2 849 565   (OLYMPUS OPTICAL CO.)<br>*Anspruch 1; Spalten 1-3* | 1,2,5 | |
| | --- | | |
| A | US-A-3 694 317   (S.SCHER)<br>*Insgesamt* | 1 | |
| | --- | | |
| A | US-A-4 038 151   (N.FADLER) | | |
| | --- | | |
| A | DE-A-2 803 044   (PFIZER INC.) | | |
| | ----- | | |

| RECHERCHIERTE SACHGEBIETE (Int. Cl. 3) |
|---|
| C 12 Q<br>C 12 K<br>C 12 M |

Der vorliegende Recherchenbericht wurde für alle Patentansprüche erstellt.

| Recherchenort<br>DEN HAAG | Abschlußdatum der Recherche<br>25-10-1982 | Prüfer<br>OSBORNE H.H. |
|---|---|---|